(19) Europäisches Patentamt
European Patent Office
Office européen des brevets

(11) Publication number: 0 190 786 B1

(12) # EUROPEAN PATENT SPECIFICATION

(45) Date of publication of patent specification: 04.04.90

(51) Int. Cl.$^5$: C 07 D 205/08, C 07 D 205/04 // A01N43/44

(21) Application number: 86200091.6

(22) Date of filing: 20.01.86

(54) **Azetidine derivatives; process for their preparation; and their use as intermediates.**

(30) Priority: 08.02.85 GB 8503193

(43) Date of publication of application: 13.08.86 Bulletin 86/33

(45) Publication of the grant of the patent: 04.04.90 Bulletin 90/14

(84) Designated Contracting States: AT BE CH DE FR GB IT LI LU NL SE

(56) References cited:
EP-A-0 114 079
EP-A-0 140 437
DE-A-2 700 178
GB-A-2 140 003

THE JOURNAL OF ORGANIC CHEMISTRY, vol. 37, no. 24, 1972 A.G. ANDERSON, R. LOK, "The Synthesis of Azetidine-3-carboxylic Acid" pages 3953-3955

(73) Proprietor: SHELL INTERNATIONALE RESEARCH MAATSCHAPPIJ B.V.
Carel van Bylandtlaan 30
NL-2596 HR Den Haag (NL)

(72) Inventor: Orr, Alexander Ferguson
Carel van Bylandtlaan 30
NL-2596 HR The Hague (NL)
Inventor: Clifford, David Ronald
55 Imperial Drive
Warden Bay Isle of Sheppey Kent (GB)

(74) Representative: Bennett, David Arthur Horder et al
4, York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 190 786 B1

## Description

This invention relates to certain substituted azetidine compounds, which are useful as intermediates in the preparation of biologically active azetidine compounds, together with the preparation of such intermediates.

It is known from European Patent 29265 that 3-carboxyazetidine and related compounds are chemical hybridising agents, their mode of action presumably being based on their ability to produce male sterility in plants. That application also describes a process for their preapration; other processes being described in European patent applications publication nos. 114079, 125714 and 140437. Although these processes are adequate, commercial flexibility is assisted by the availability of alternative process routs and such an alternative is provided via the novel azetidinones of the present application.

Accordingly, this invention relates in one aspect to substituted azetidinone carboxylates of the general formula I:

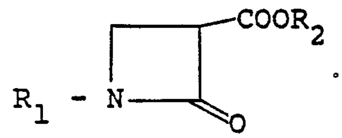

I

wherein $R_1$ represents a benzhydryl, tosyl or, preferably, a benzyl group, and $R_2$ represents a hydrogen atom or an alkyl group of up to 6 carbon atoms, preferably a methyl group.

In a further aspect, the invention relates to a process for the preparation of an azetidinone of formula I which comprises oxidising an N-substituted dioxopyrrolidine of the general formula II:

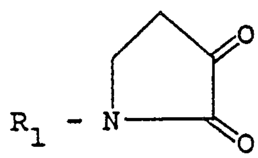

II

wherein $R_1$ is as defined above. The oxidation, which brings about a contraction from a 5-membered to a 4-membered ring, is most conveniently brought about by a perhalate, such as perchlorate or periodate, suitably used as an alkali metal salt, the preferred oxidising agent being sodium periodate. This reaction is desirably carried out under substantially neutral conditions (i.e. pH7), and preferably in the presence of a buffer which enables that desired pH to be attained and maintained during the reaction. Any buffering agent which does not interfere with the actual oxidation reaction may be used; a sodium phosphate buffer being one example of a suitable buffer system. The oxidation reaction may be effected at any convenient temperature, e.g. above 0°C (when reaction is slow) but below temperatures at which thermal decomposition competes with the desired oxidation reaction (e.g. 60°C); satisfactory results are generally achieved at ambient temperature (e.g. 15—25°C).

The azetidinone carboxylates of formula I are useful intermediates. In particular, they may be used as intermediates in the preparation of 3-hydroxymethyl azetidine derivatives, which compounds themselves may be used in the preparation of azetidine-3-carboxylic acid derivatives which exhibit plant growth regulant properties, especially the property of rendering sterile the male parts of plants. Thus, in a further aspect, the invention includes also a process for the preparation of an N-substituted-3-hydroxymethyl azetidine of general formula III:—

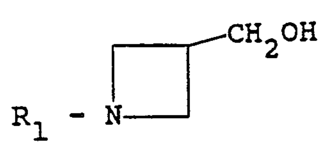

III

which comprises reduction of an N-substituted azetidinone of formula I above, wherein $R_1$ and $R_2$ are as defined in respect of formula I. These compoounds of formula I wherein $R_2$ represents an alkyl group are, of course, esters, which may be prepared from the corresponding free acid (wherein $R_2$ represents a hydrogen atom) by any of the well-established synthetic esterification procedures. Thus, if the methyl ester is required, this may conveniently be prepared from the free acid by reaction with diazomethane (or the adduct 'diazald®'). The reduction of the azetidinone may be carried out by any suitable reducing agent; convenient agents being hydrides such as lithium aluminium (or boron) hydride or, preferably, borane, used in an inert solvent such as tetrahydrofuran. This reduction may be carried out at any temperature between −50°C and the reflux temperature of the reaction mixture, but for reasons of operating convenience is suitably carried out at room temperature. The resulting 3-hydroxymethyl azetidine of formula III may then, if required, be converted to azetidine-3-carboxylic acid by catalytic oxidation followed by catalytic hyrogenation, using convention procedures. The invention accordingly further includes the use of azetidinones of formula I, and the 3-hydroxymethyl azetidines prepared therefrom, as intermediates for the preparation of azetidine-3-carboxylic acid derivatives.

The invention is illustrated in the following Examples.

### Example 1

Preparation of 1-Benzyl-3-carboxy-azetidin-2-one.

1-Benzyl-2,3-dioxopyrrolidine (57.3 g; prepared according to the procedure described in J. Am. Chem. Soc. *75*, 3413 (1953)) was added to 5000 ml of a 0.2M sodium phosphate buffer, sodium periodate (390 g) added, and the reaction mixture stirred in the dark for 24 hours. (The buffer had been prepared by adding 5M aqueous sodium hydroxide solution to 2 litre of 0.5M orthophosphoric acid until the pH was 7.0. This solution was then diluted to a volume of 5 litre with distilled water).

The reaction mixture was then worked up by the slow addition, with ice-bath cooling, of 2M aqueous sodium bisulphite. The pH was kept near 7.0 by addition of saturated potassium carbonate solution during the addition of bisulphite, and then acidified to pH 4.0 with phosphoric acid. The mixture was extracted with dichloromethane to yield a crude product, which was chromatographically purified to yield the title product as an oil. The material was characterised by its n.m.r. characteristics:—

delta values in $CDCl_3$:—

3.4 (2H.m. $CH_2$—N)
4.1 (1H.m. $CH$—CO)
4.5 (2H.s. $CH_2$ Aryl)
7.2 (5H.s. aryl $H$)
11.0 (1H broad $CO_2H$)

### Example 2

Preparation of 1-Benzyl-3-methoxycarbonyl-azetidin-2-one

Potassium hydroxide (2.47 g), water (4 ml) and 2-methoxyethanol (10 ml) were placed into a flask which was immersed in cold water. p-Tolylsulphonylmethyl nitrosamide (8.85 g) was dissolved in ether (50 ml), and this was added to the flask, which was then stopped with a bung pierced by a glass tube. The other end of the tube was placed in a second flask containing 15 ml ether, with its tip below the surface of the ether. The second flask was cooled in an ice-bath and the first gently warmed until the yellow colour had disappeared from the first flask. Heating was stopped and to the second flask was slowly added 1-benzyl-3-carboxy-azetidin-2-one (5 g, prepared as in Example 1) dissolved in ether/methanol.

The second flask was shaken and left standing at room temperature for 15 hours, after which excess diazomethane was destroyed by dropwise addition of acetic acid until effervescence ceased. The solvent was evaporated off, and the title product recovered as an oil, characterised by its n.m.r. (and mass spectrum):

delta values in $CCl_4$:

3.5 (2H.m. $CH_2$—N)
3.9 (3H.s. $OCH_3$)
4.2 (1H.m. $CH$—CO)
4.6 (2H.s. $CH_2$-aryl)
7.3 (5H-aryl$H$)

### Example 3

Preparation of 1-Benzyl-3-hydroxymethyl azetidine

A 1 molar solution of diborane in tetrahydrofuran (30 ml) was cooled to 0°C under nitrogen, and to this was added dropwise a solution of 1-benzyl-3-methoxycabronyl-azetidin-2-one (4.23 g, obtained as in Example 2) in tetrahydrofuran (20 ml). When addition was complete, the mixture was stirred at room temperature for 30 minutes, and then at 50°C for 2 hours. The solution was then cooled, and maintained between 5—10°C during the dropwise addition of a 10% sodium hydroxide solution (16 ml). The mixture was stirred at room temperature for 30 minutes, the organic layer separated, and the aqueous layer washed twice with ether. The organic layers were combined, dried with anhydrous magnesium sulphate, and the solvent removed to yield a crude product, which was purified chromatographically to the title product, characterised by its n.m.r.:—

delta values in $CDCl_3$

3.1 (1H.m. ring $CH$)
3.38 (2H.d. $CH_2$OH)
3.55 (t.2H. ring $CH_2$)
3.68 (t.2H ring $CH_2$)
3.91 (2H.s. $CH_2$ aryl)
7.40 (5H.s. aryl $H$)

### Example 4

Use of 1-benzyl-3-hydroxymethylazetidine to prepare azetidine-3-carboxylic acid

1-Benzyl-3-hydroxymethylazetidine (17.7 g, prepared as in Example 3) and sodium hydroxide (4.0 g) in water (350 ml) were stirred at 80°C with a 5% platinum on charcoal catalyst (7.0 g) while a stream of oxygen was passed into the mixture for two hours. The cooled mixture was then filtered and the water was removed from the filtrate under reduced pressure. The residue was taken up in acetic acid (100 ml) and hydrogenated in the presence of a 5% palladium on charcoal catalyst (2.4 g). The mixture was filtered and the filtrate was evaporated. The solid residue was dissolved in water and passed down a "Dowex" ion

exchange column using 2 M ammonium hydroxide solution as eluent. "Dowex" is a registered Trade Mark. Fractions giving a positive ninhydrin test were evaporated to give azetidine-3-carboxylic acid as a white crystalline solid, m.p. 285—290°C (dec), yield 70%.

**Claims for the Contracting States: BE CH DE FR GB IT LI LU NL SE**

1. Substituted azetidinone carboxylates of the general formula I

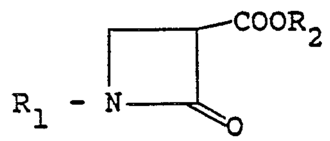

I

wherein $R_1$ represents a benzyl, benzhydryl or tosyl group and $R_2$ represents a hydrogen atom or an alkyl group of up to 6 carbon atoms.

2. Compounds as claimed in claim 1 wherein $R_1$ represents a benzyl group and $R_2$ represents a hydrogen atom or a methyl group.

3. Process for the preparation of azetidinones as defined in claim 1, which comprises oxidising an N-substituted dioxopyrrolidine of general formula II:—

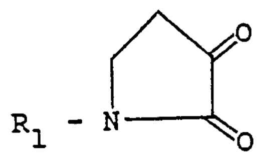

II

wherein $R_1$ is as defined in claim 1.

4. Process as claimed in claim 3 wherein the oxidation reaction is effected under substantially neutral conditions.

5. Process as claimed in claim 3 or 4 wherein the oxidation is effected by reaction with an alkali metal periodate, and in the presence of a buffer.

6. Process as claimed in any one of claims 3—5 wherein the azetidinone carboxylate product is reduced to an N-substituted-3-hydroxymethyl azetidine derivative of the formula III

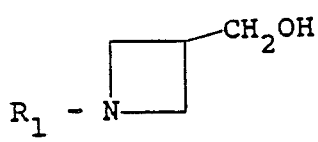

III

7. Process as claimed in claim 6 wherein the reduction is effected by reaction with borane in an inert organic solvent.

8. Use of a substituted azetidinone carboxylate as defined in claim 1 as an intermediate in the preparation of an N-substituted-3-hydroxymethyl azetidine of formula III.

9. Process for the preparation of an N-substituted-3-hydroxymethyl azetidine derivative of general formula III

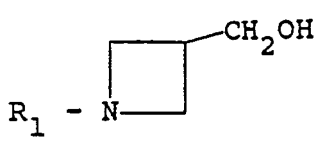

III

which comprises reduction of an N-substituted azetidinone carboxylate of general formula I:

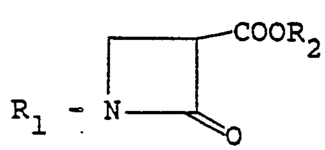

I

wherein $R_1$ and $R_2$ are as defined in claim 1.

10. Process as claimed in claim 9 wherein the reduction is effected with borane in the presence of an inert solvent.

11. Process as claimed in claim 10 wherein the inert solvent is tetrahydrofuran.

12. Process as claimed in any one of claims 9—11 wherein the N-substituted azetidinone carboxylate reactant of formula I is prepared by oxidation of an N-substituted dioxopyrrolidine of general formula II:

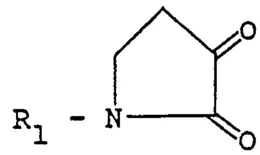

II

wherein $R_1$ is as defined in claim 1.

13. Process as claimed in claim 12 wherein the oxidation is effected by reaction with an alkali metal periodate under substantially neutral conditions.

14. Use of an azetidine derivative as defined in claim 1 or 2 as an intermediate for the preparation of azetidine-3-carboxylic acid.

**Claims for the Contracting State: AT**

1. Process for the preparation of azetidinones of the general formula I

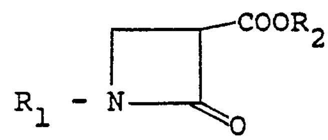

I

wherein $R_1$ represents a benzyl, benzhydryl or tosyl group and $R_2$ represents a hydrogen atom or an alkyl group of up to 6 carbon atoms, which comprises oxidising an N-substituted dioxopyrrolidine of general formula II:—

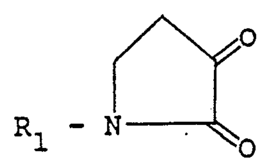

II

wherein $R_1$ is as defined above.

2. Process as claimed in claim 1 wherein, in formula I, $R_1$ represents a benzyl group and $R_2$ represents a hydrogen atom or a methyl group.

3. Process as claimed in claim 1 or 2 wherein the oxidation reaction is effected under substantially neutral conditions.

4. Process as claimed in claim 1—3 wherein the oxidation is effected by reaction with an alkali metal periodate, and in the presence of a buffer.

5. Process as claimed in any one of claims 1—4 wherein the azetidinone carboxylate product is reduced to an N-substituted-3-hydroxymethyl azetidine derivative of the formula III

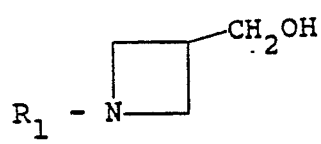

III

6. Process as claimed in claim 5 wherein the reduction is effected by reaction with borane in an inert organic solvent.

7. Use of a substituted azetidinone carboxylate as defined in claim 1 as an intermediate in the preparation of an N-substituted-3-hydroxymethyl azetidine of formula III.

8. Process for the preparation of an N-substituted-3-hydroxymethyl azetidine derivative of general formula III

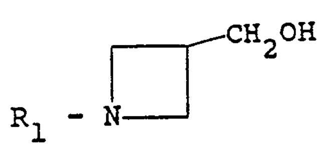

III

which comprises reduction of an N-substituted azetidinone carboxylate of general formula I:

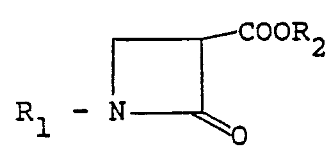

I

wherein $R_1$ and $R_2$ are as defined in claim 1.

9. Process as claimed in claim 8 wherein the reduction is effected with borane in the presence of an inert solvent.

10. Process as claimed in claim 9 wherein the inert solvent is tetrahydrofuran.

11. Process as claimed in any one of claims 6—10 wherein the N-substituted azetidinone carboxylate reactant of formula I is prepared by oxidation of an N-substituted dioxopyrrolidine of general formula II:

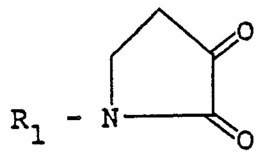

II

wherein $R_1$ is as defined in claim 1.

12. Process as claimed in claim 11 wherein the oxidation is effected by reaction with an alkali metal periodate under substantially neutral conditions.

13. Use of an azetidine derivative as defined in claim 1 or 2 as an intermediate for the preparation of azetidine-3-carboxylic acid.

**Patentansprüche für die Vertragsstaaten: BE CH DE FR GB IT LI LU NL SE**

1. Substituierte Azetidinoncarboxylate der allgemeinen Formel I

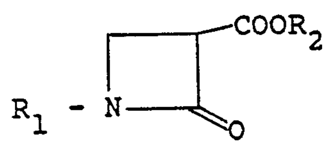

I

worin $R_1$ eine Benzyl-, Benzhydryl- oder Tosylgruppe bedeutet und $R_2$ ein Wasserstoffatom oder eine Alkylgruppe mit bis zu 6 Kohlenstoffatomen darstellt.

2. Verbindungen nach Anspruch 2, worin $R_1$ eine Benzylgruppe bedeutet und $R_2$ ein Wasserstoffatom oder eine Methylgruppe darstellt.

3. Verfahren zur Herstellung von Azetidinonen, wie in Anspruch 1 definiert, welches ein Odieren eines N-substituierten Dioxopyrrolidins der allgemeinen Formel II:

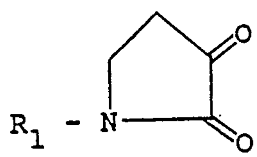

II

worin $R_1$ wie in Anspruch 1 definiert ist, umfaßt.

4. Verfahren nach Anspruch 3, worin die Oxidationsreaktion unter im wesentlichen neutralen Bedingungen ausgeführt wird.

5. Verfahren nach Anspruch 3 oder 4, worin die Oxidation durch Umsetzung mit einem Alkalimetallperiodat und in Anwesenheit eines Puffers ausgeführt wird.

6. Verfahren nach einem der Ansprüche 3 bis 5, worin das Azetidinoncarboxylatprodukt zu einem N-substituierten 3-Hydroxymethylazetidinderivat der Formel III:

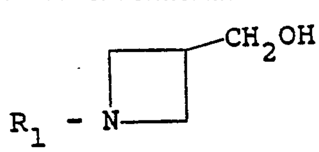

III

reduziert wird.

7. Verfahren nach Anspruch 6, worin die Reduktion durch Umsetzung mit Boran in einem inerten organischen Lösungsmittel ausgeführt wird.

8. Verwendung eines substituierten Azetidinoncarboxylats, wie in Anspruch 1 definiert, als Zwischenprodukt in der Herstellung eines N-substituierten 3-Hydroxymethylazetidins der Formel III.

9. Verfahren zur Herstellung eines N-substituierten 3-Hydroxymethylazetidinderivats der allgemeinen Formel III:

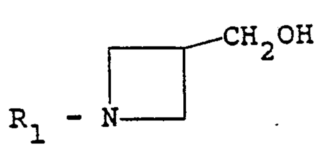

III

welches ein Reduzieren eines N-substituierten Azetidinoncarboxylats der allgemeinen Formel I:

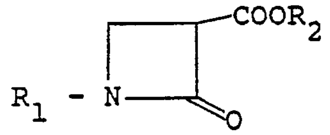

I

worin $R_1$ und $R_2$ wie in Anspruch 1 definiert sind, umfaßt.

10. Verfahren nach Anspruch 9, worin die Reduktion mit Boran in Anwesenheit eines inerten Lösungsmittels ausgeführt wird.

11. Verfahren nach Anspruch 10, worin das inerte Lösungsmittel Tetrahydrofuran ist.

12. Verfahren nach einem der Ansprüche 9 bis 11, worin das N-substituierte Azetidinoncarboxylat-Reagens der Formel I durch Oxidation eines N-substituierten Dioxopyrrolidins der allgemeinen Formel II:

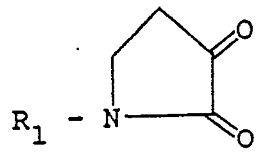

II

worin $R_1$ wie in Anspruch 1 definiert ist, hergestellt wird.

13. Verfahren nach Anspruch 12, worin die Oxidation durch Umsetzung mit einem Alkalimetallperiodat unter im wesentlichen neutralen Bedingungen ausgeführt wird.

14. Verwendung eines Azetidinderivats, wie in Anspruch 1 oder 2 definiert, als Zwischenprodukt für die Herstellung von Azetidin-3-karbonsäure.

**Patentansprüche für den Vertragsstaat: AT**

1. Verfahren zur Herstelung von Azetidinonen der allgemeinen Formel I

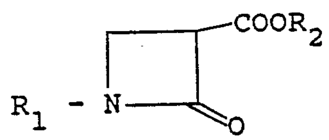

I

worin $R_1$ eine Benzyl-, Benzhydryl- oder Tosylgruppe bedeutet und $R_2$ ein Wasserstoffatom oder eine Alkylgruppe mit bis zu 6 Kohlenstoffatomen darstellt, welches ein Oxidieren eines N-substituierten Dioxopyrrolidins der allgemeinen Formel II

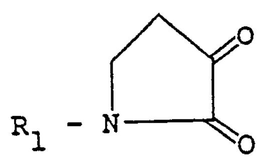

II

worin $R_1$ wie oben definiert ist, umfaßt.

2. Verfahren nach Anspruch 1, worin in Der Formel I $R_1$ eine Benzylgruppe bedeutet und $R_2$ ein Wasserstoffatom oder eine Methylgruppe darstellt.

3. Verfahren nach Anspruch 1 oder 2, worin die Oxidationsreaktion unter im wesentlichen neutralen Bedingungen ausgeführt wird.

4. Verfahren nach den Ansprüchen 1 bis 3, worin die Oxidation durch Umsetzung mit einem Alkalimetallperiodat und in Anwesenheit eines Puffers ausgeführt wird.

5. Verfahren nach einem der Ansprüche 1 bis 4, worin das Azetidinoncarboxylatprodukt zu einem N-substituierten 3-Hydroxymethylazetidinderivat der Formel III:

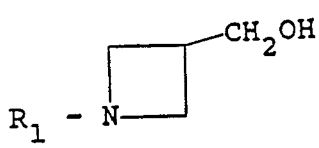

III

reduziert wird.

6. Verfahren nach Anspruch 5, worin die Reduktion durch Umsetzung mit Boran in einem inerten organischen Lösungsmittel ausgeführt wird.

7. Verwendung eines substituierten Azetidinoncarboxylats, wie in Anspruch 1 definiert, als Zwischenprodukt in der Herstellung eines N-substituierten 3-Hydroxymethylazetidins der Formel III.

8. Verfahren zur Herstellung eines N-substituierten 3-Hydroxymethylazetidinderivats der allgemeinen Formel III:

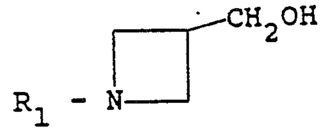

III

welches ein Reduzieren eines N-substituierten Azetidinoncarboxylats der allgemeinen Formel I:

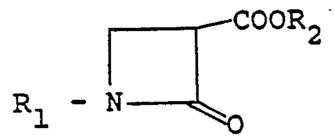

I

worin $R_1$ und $R_2$ wie in Anspruch 1 definiert sind, umfaßt.

9. Verfahren nach Anspruch 8, worin die Reduktion mit Boran in Anwesenheit eines inerten Lösungsmittels ausgeführt wird.

10. Verfahren nach Anspruch 9, worin das inerte Lösungsmittel Tetrahydrofuran ist.

11. Verfahren nach einem der Ansprüche 6 bis 10, worin das N-substituierte Azetidinoncarboxylat-Reagens der Formel I durch Oxidation eines N-substituierten Dioxopyrrolidins der allgemeinen Formel II:

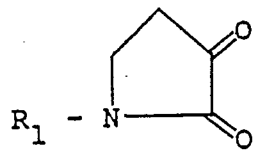

II

worin $R_1$ wie in Anspruch 1 definiert ist, hergestellt wird.

12. Verfahren nach Anspruch 11, worin die Oxidation durch Umsetzung mit einem Alkalimetallperiodat unter im wesentlichen neutralen Bedingungen ausgeführt wird.

13. Verwendung eines Azetidinderivats, wie in Anspruch 1 oder 2 definiert, als Zwischenprodukt für die Herstellung von Azetidin-3-karbonsäure.

**Revendications pour les Etats contractants: BE CH DE FR GB IT LI LU NL SE**

1. Carboxylate d'azétidinone substituée de la formule générale I

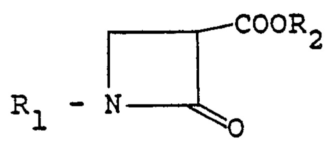

I

dans laquelle $R_1$ représente un groupe benzyle, benzhydryle ou tosyle et $R_2$ représente un atome d'hydrogène ou un groupe alkyle allant jusqu'à 6 atomes de carbone.

2. Composés tels que revendiqués dans la revendication 1, dans lesquels $R_1$ représente un groupe benzyle et $R_2$ représente un atome d'hydrogène ou un groupe méthyle.

3. Procédé pour la préparation d'azétidinones telles que définies dans la revendication 1, qui consiste à oxyder une dioxopyrrolidine N-substituée de formule générale II:

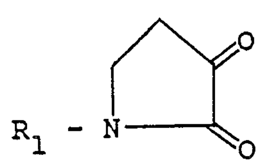

II

dans laquelle $R_1$ est tel que défini dans la revendication 1.

4. Procédé tel que revendiqué dans la revendication 3, dans lequel la réaction d'oxydation est réalisée sous des conditions pratiquement neutres.

5. Procédé tel que revendiqué dans la revendication 3 ou 4, dans laquelle l'oxydation est réalisée par réaction avec un periodate de métal alcalin, et en présence d'un tampon.

6. Procédé tel que revendiqué dans l'une quelconque des revendications 3 à 5, dans lequel le produit carboxylate d'azétidinone est réduit en un dérivé de 3-hydroxyméthyl-azétidine n-substituée de la formule III:

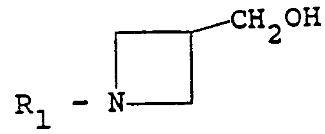

III

7. Procédé tel que revendiqué dans la revendication 6, dans lequel la réduction est réalisée par réaction avec du borane dans un solvant organique inerte.

8. Utilisation d'un carboxylate d'azétidinone substituée tel que défini dans la revendication 1 comme un intermédiaire dans la préparation de la 3-hydroxyméthyl-azétidine N-substituée de formule III.

9. Procédé pour la préparation d'un dérivé de 3-hydroxyméthyl-azétidine N-substituée de formule générale III:

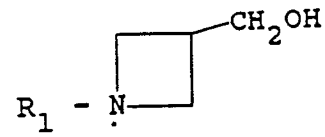

III

qui consiste à réduire un carboxylate d'azétidinone N-substituée de formule générale I:

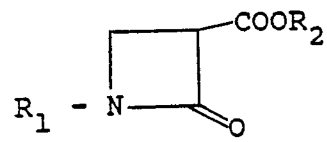

I

dans laquelle $R_1$ et $R_2$ sont tels que définis dans la revendication 1.

10. Procédé tel que revendiqué dans la revendication 9, dans lequel la réduction est réalisée avec du borane en présence d'un solvant inerte.

11. Procédé tel que revendiqué dans la revendication 10, dans lequel le solvant inerte est le tétrahydrofurane.

12. Procédé tel que revendiqué dans l'une quelconque des revendications 9 à 11, dans lequel le réactif carboxylate d'azétidinone N-substituée de la formule I est préparé par oxydation d'une dioxopyrrolidine N-substituée de formule générale:

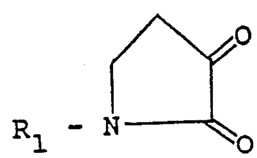

II

dans laquelle $R_1$ est tel que défini dans la revendication 1.

13. Procédé tel que revendiqué dans la revendication 12, dans lequel l'oxydation est réalisée par réaction avec un periodate de métal alcalin sous des conditions pratiquement neutres.

14. Utilisation d'un dérivé d'azétidine tel que défini dans la revendication 1 ou 2 comme un intermédiaire pour la préparation de l'acide azétidine-3-carboxylique.

**Revendications pour l'Etat contractant: AT**

1. Procédé pour la préparation d'azétidinones de formule générale I

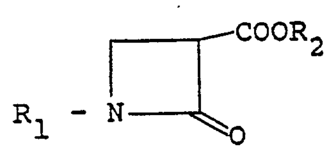

I

dans laquelle $R_1$ représente un groupe benzyle, benzhydryle ou tosyle et $R_2$ représente un atome d'hydrogène ou un groupe alkyle allant jusqu'à 6 atomes de carbone, qui consiste à oxyder une dioxopyrrolidine N-substituée de formule générale

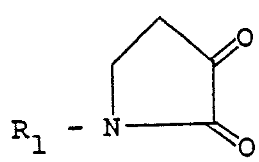

II

dans laquelle $R_1$ est tel que défini ci-dessus.

2. Procédé tel que revendiqué dans la revendication 1, dans lequel, dans la formule I, $R_1$ représente un groupe benzyle et $R_2$ représente un atome d'hydrogène ou un groupe méthyle.

3. Procédé tel que revendiqué dans la revendication 1 ou 2, dans lequel la réaction d'oxydation est realisée sous des conditions pratiquement neutres.

4. Procédé tel que revendiqué dans la revendication 1 à 3, caractérisé en ce que l'oxydation est réalisée par réaction avec un periodate de métal alcalin, et en prèsence d'un tampon.

5. Procédé tel que revendiqué dans l'une quelconque des revendications 1 ou 4, dans lequel le produit carboxylate d'azétidinone est réduit en un dérivé de 3-hydroxyméthyl-azétidine n-substitué de la formule III:

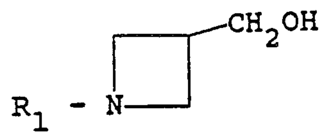

III

6. Procédé tel que revendiqué dans la revendication 5, dans lequel la réduction est réalisée par réaction avec du borane dans un solvant organique inerte.

7. Utilisation d'un carboxylate d'azétidinone substituée tel que défini dans la revendication 1 comme un intermédiaire dans la préparation d'une 3-hydroxyméthyl-azétidine N-substituée de formule III.

8. Procédé pour la préparation d'un dérivé de 3-hydroxyméthyl-azétidine N-substituée de formule générale III:

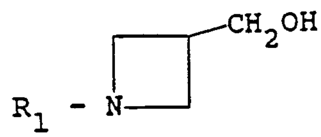

III

qui consiste à réduire un carboxylate d'azétidinone N-substituée de formule générale I:

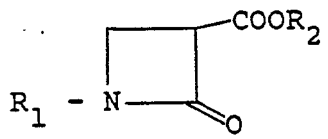

I

dans laquelle $R_1$ et $R_2$ sont tels que définis dans la revendication 1.

9. Procédé tel que revendiqué dans la revendication 8, dans lequel la réduction est réalisée avec du borane en présence d'un solvant inerte.

10. Procédé tel que revendiqué dans la revendication 9, dans lequel le solvant inerte est le tétrahydrofurane.

11. Procédé tel que revendiqué dans l'une quelconque des revendications 6 à 10, dans lequel le réactif carboxylate d'azétidinone N-substituée de la formule I est préparé par oxydation d'une dioxopyrrolidine N-substituée de formule générale II:

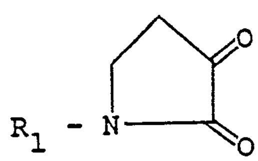

II

dans laquelle $R_1$ est tel que défini dans la revendication 1.

12. Procédé tel que revendiqué dans la revendication 11, dans lequel l'oxydation est réalisée par réaction avec un periodate de métal alcalin sous des conditions pratiquement neutre.

13. Utilisation d'un dérivé d'azétidine tel que défini dans la revendication 1 ou 2 comme un intermédiaire pour la préparation d'un l'acide azétidine-3-carboxylique.